# EUROPEAN PATENT APPLICATION

(11) **EP 1 595 542 A1**
(43) Date of publication of application: **16.11.2005**
(21) Application number: 04101253.5
(22) Date of filing: 26.03.2004
(51) Int. Cl.: A61K 31/496, A61P 25/16, A61P 25/14

(54) **Transdermal iontophoretic delivery of piperazinyl-2(3H)-benzoxazolone compounds**

(71) Applicant: Solvay Pharmaceuticals B.V., 1381 CP Weesp (NL); LEIDEN UNIVERSITY, 2300 RA Leiden (NL)
(72) Inventor: Van den Berg, Dirk-Jan, 2300 RA, LEIDEN (NL); Bouwstra, Johanna A., 2300 RA, LEIDEN (NL); Verbaan, Frederik J., 2300 RA, LEIDEN (NL)
(74) Representative: Verhage, Marinus

(57) **Abstract**

The present invention is related to the use of a compound of the general formula 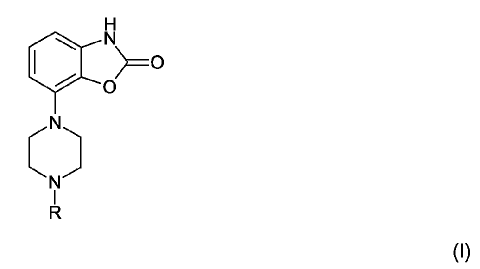 wherein R is methyl, ethyl, ethyl substituted with one or more fluorine atoms, or cycloalkyl-(C₃-C₇)-methyl optionally substituted with one or more fluorine atoms or benzyl optionally substituted with a group selected from phenyl, furanyl and thienyl which groups are optionally substituted with 1-3 substituents from the group hydroxy, halogen, C₁₋₄-alkoxy, C₁₋₄-alkyl, cyano, aminocarbonyl, mono- or di-C₁₋₄-alkylaminocarbonyl; and pharmaceutically acceptable salts and prodrugs thereof, for the manufacture of a composition for transdermal administration by iontophoresis.

## Description

### FIELD OF THE INVENTION

The present invention relates to transdermal iontophoretic delivery of pharmaceutical compounds of the general formula 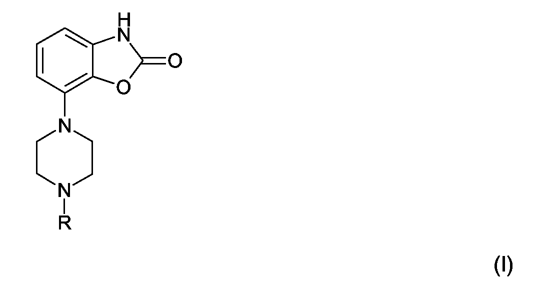 wherein R is methyl, ethyl, ethyl substituted with one or more fluorine atoms, or cycloalkyl-(C₃ - C₇) - methyl optionally substituted with one or more fluorine atoms or benzyl optionally substituted with a group selected from phenyl, furanyl and thienyl which groups are optionally substituted with 1-3 substituents from the group hydroxy, halogen, C₁₋₄-alkoxy, C₁₋₄-alkyl, cyano, aminocarbonyl, mono- or di-C₁₋₄-alkylaminocarbonyl; and pharmaceutically acceptable salts and prodrugs thereof.

More specifically, the present invention relates to the use of compounds of the general formula (I) for the preparation of (a) a solution for use in a patch for transdermal administration by iontophoresis or kits containing cartridges which contain the compound ready for use in said patch, (b) a patch suitable for transdermal administration by iontophoresis, wherein said transdermal patch has a reservoir containing the compound of formula I or a composition thereof and optionally a pharmaceutically acceptable electrolyte, whi ch patch can be used in a method for controlling the delivery profile of pharmaceutical compounds of the general formula (I) and compositions thereof, and the use of said controlled delivery profiles in the treatment of pain disorders, especially restless leg syndrome and CNS disorders, especially Parkinson's disease. R is preferably selected from the group consisting of methyl, benzyl optionally substituted with a phenyl group having 1-3 substituents from the group hydroxyl and halogen. Most preferred comp ounds are compounds wherein R is methyl or benzyl.

### BACKGROUND OF THE INVENTION

Compounds of the general formula I as defined above are known from WO00/29397 and WO01/85725. These compounds show varying activities as either partial agonists or agonists at the dopamine D₂ receptor and are also agonists of the 5HT1 _{A} receptor. These combinations of activities make the compounds of value for the treatment of affections or diseases of the central nervous system caused by disturbances in either the dopaminergic or serotinergic systems, especially in Parkinson's disease and restless leg syndrome.

In certain cases, e.g. when oral delivery or injection of a particular pharmaceutical compound may be ineffective or unacceptable because of poor gastrointestinal absorption, an extensive first pass effect, patient pain and discomfort, or other side effects or drawbacks, transdermal delivery may provide an advantageous method of delivering that compound. This is e.g. the case for Parkinson's disease, where there is a need to administer medication to patients who are sleeping, comatose or anaesthetized. Further there is growing evidence that continuous dopamine stimulation avoids the development of problems associated with intermittent dosing and where continuous drug delivery has been shown to decrease the incidence of "off" periods (P. Niall and W.H. Oertel, Congress Report of 7^{th} International Congress of Parkinsons's Disease and Movement Disorders, Miami, Florida, November 10 -14, 2002). In general transdermal administration also has its problems, since it is not easy to get drugs to cross the skin.

Iontophoretic transdermal delivery relates to introducing ions or soluble salts of pharmaceutically active compounds into tissues of the body under the influence of an applied electric field.
The features and benefits of iontophoretic transdermal delivery systems as compared with passive transdermal systems, as well as with other means of delivering pharmaceutical compounds into the bloodstream have e.g. been reviewed in O. Wong, "Iontophoresis: Fundamentals," in Drugs Pharm. Sci. (1994), 62 (Drug Permeation Enhancement), 219-46 (1994); P.Singh et al., "Iontophoresis in Drug Delivery : Basic Principles and Applications", Critical Reviews in Therapeutic Drug Carrier Systems, 11 (2 & 3) : 161-213 (1994); and Ajay K. Banga, Electrically Assisted Transdermal and Topical Drug Delivery, Taylor and Francis Group Ltd., London UK, 1998, ISBN 0-7484-0687-5.

In certain cases, e.g. when transdermal delivery by means of patches appears to be ineffective or unacceptable because of low passage through the skin, leading to very large patches, iontophoretic transdermal delivery may provide an advantageous method of delivering that compound. Further iontophoretic transdermal delivery has the major advantage that the administered amount can be regulated precisely and can be used to easily titrate patients up to a certain level of administration over a period up to several weeks.

Despite these advantages, iontophoretic methods appear limited as the drug delivery profile of a particular method depends heavily on the particular drug administered. Generally, once it has been established that a specific drug may be effectively delivered by iontophoresis, success in adjusting the delivery profile of that drug is not always guaranteed. Although a lot of experiments have been done with the iontophoretic delivery of various active substances, specific information allowing a person skilled in the art to tailor the delivery profile of a specific drug is not available.

As it has appeared that it is very difficult to develop transdermal patches with an acceptable size for compounds with the general formula (I), there is a need for an iontophoretic delivery method for said that allows variable rate delivery of said compounds tailored to its specific treatment.

### SUMMARY OF THE INVENTION

It is an objective of the present invention to provide iontophoretic transdermal technology that provides the delivery of the compounds of the general formula (I) and compositions thereof through the human skin.

More specifically it is an object of the present invention to provide for the use of a compound of the general formula (I) and pharmaceutically acceptable salts and prodrugs thereof for the manufacture of a composition sui table for use in a patch for transdermal administration by iontophoresis, wherein said composition comprises the compound of formula I and optionally a pharmaceutically acceptable electrolyte.

Still more specifically, an object of the subject invention is to provide the use of the compounds of the general formula (I) and pharmaceutically acceptable salts and prodrugs thereof for the manufacture of a patch suitable for transdermal administration by iontophoresis, wherein said transdermal patch has a reservoir containing the compound of formula I or a composition thereof and optionally a pharmaceutically acceptable electrolyte. When this patch is applied to the skin of a living body and electrical current is caused to flow through the skin, the compounds of the general formula (I) and pharmaceutically acceptable salts and prodrugs thereof are iontophoretically delivered through the skin.

Another object of the invention is to provide an iontophoretic system for the delivery of the compounds of the general formula (I) and compositions thereof through the skin, wherein the system includes a transdermal delivery device attachable to the skin, the device including a first electrode and a second electrode, and a reservoir for containing a pharmaceutically acceptable electrolyte and the compounds of the general formula (I) and compositions thereof in electrical communication with the first and second electrodes; and an electrical power source connected to the first and second electrodes; wherein the reservoir contain s the compounds of the general formula (I) and compositions thereof and optionally a pharmaceutically acceptable electrolyte.

It is also an object of the invention to provide a kit comprising the iontophoretic system combined with one or more cartridges comprising the compound of the general formula (I) or a kit containing one or more cartridges comprising the compound of the general formula (I) to be used for refilling the reservoir of the iontophoretic system. The amount of cartridges in the kits is preferably between 2 and 28 and most preferably between 7 and 14.

The skin through which the delivery has to take place is animal skin, preferably human skin.

### BRIEF DESCRIPTION OF THE FIGURES

Figure 1 plots the flux of 7-(4-methyl-1-piperazinyl-2(3H)-benzoxazolone across human stratum corneum as a function of the active compound concentration versus time.
Figure 2 plots the flux of 7-(4-methyl-1-piperazinyl-2(3H)-benzoxazolone across human stratum corneum as a function of the electrolyte concentration versus time.
Figure 3 plots the flux of 7-(4-methyl-1-piperazinyl-2(3H)-benzoxazolone across human stratum corneum as a function of active compound concentration versus time in the presence of 4 g/I NaCl.
Figure 4 depicts the schematic presentation of the iontophoretic set-up.

### DETAILED DESCRIPTION OF INVENTION

An iontophoretic transdermal delivery system typically includes a first (donor) electrode containing an electrolytically available active compound within a suitable vehicle or carrier and optionally a penetration enhancer, a counter electrode and a power source, the first and second electrodes each being in electrically conductive communication with the power source. The first and second electrodes are being adapted for spaced apart physical contact with the skin whereby, in response to a current provided by the power source through the electrodes, a therapeutic amount of the active compound is administered through the skin to a patient.

It has surprisingly been found that the iontophoretic delivery (dose and profile) by which a particular active compound of the general formula (I) is administered to a patient may be very well controlled by suitable combination of the initial concentration of the drug and electrolyte and the applied current (constant/variable) in the iontophoretic system. More specifically, it has been found that the combination of current density (constant/variable) and the initial amount of electrolyte leads to an iontophoretic patch with a very reasonable size that allows the drug delivery profile to be adjusted. The ability to tailor the drug delivery profile in iontophoresis provides increased control of the drug's effects on the user. Additionally, the ability to tailor drug delivery profile in iontophoresis makes the iontophoretic delivery of the compounds of formula (I) to be a more practically effective mode of administration.

As used herein, the term "permeation profile" means a plot of the flux of the active compound versus time for a given delivery period.

As used herein, the term "cartridge" means a packaging containing the active compound that meets all requirements relating to storage and user-friendliness and that is used for storage of the active compound before it is filled into the patch.

The electrolyte used in the present invention method may include univalent or divalent ions. Examples of electrolytes used in our method include all Cl⁻ donating compounds that are water soluble, such as NaCl, CaCl₂, or MgCl₂. A preferred electrolyte is NaCl. The required amount of electrolyte depends on the transport area of the patch, the volume of the vehicle or carrier, the concentration of the active compound, the current density, the duration of the iontophoresis and the efficiency of the transport. The electrolyte may be present in amounts ranging from about 0.005 to about 2 mmole, preferably about 0.01 to about 1.0 mmole, more preferably about 0.05 to about 0.3 mmole. The initial amount of electrolyte may be expressed as a concentrations ranging from about 0.005 to about 2 M, preferably about 0.01 to about 0.2 M, more preferably about 0.05 to about 0.2 M.

The compounds that may be administered were already defined above. Prodrugs, are compounds which when administered to humans by any known route, are metabolised to compounds having formula (I). In particular this relates to compounds with primary or secondary amino or hydroxy groups. Such compounds can be reacted with organic acids to yield compounds having formula (I) wherein an additional group is present which is easily removed after administration, for instance, but not limited to amidine, enamine, a Mannich base, a hydroxymethylene derivative, an O-(acyloxymethylene carbamate) derivative, carbamate, ester, amide or enaminone. A prodrug is an inactive compound, which when absorbed is converted into an active form (Medicinal Chemistry: Principles and Practice, 1994, ISBN 0 - 85186-494-5, Ed.: F. D. King, p. 216).

Typical active drugs that may be administered by the method described herein include, but are not limited to, compounds such as 7-(4-methyl-1-piperazinyl-2(3H)-benzoxazolone or its monohydrochloride (SLV308, see Drugs of the Future 2001 26, 128-32) and 7-(4-benzyl-1-piperazinyl-2(3H)-benzoxazolone or its monomesylate. A preferred compound is 7-(4-methyl-1-piperazinyl-2(3H)-benzoxazolone or its monohydrochloride.

7-(4-methyl-1-piperazinyl-2(3H)- benzoxazolone or its monohydrochloride and 7-(4-benzyl-1-piperazinyl-2(3H)- benzoxazolone or its monomesylate are especially suitable for the treatment of restless leg syndrome or Parkinson's disease.

The pH of the solution in the drug reservoir will typically range from 3.0 to 7.5, preferably 4.0 to 6.5. The pH is maintained on a constant level by means of a buffer such as a citrate buffer or a phosphate buffer.
For 7-(4-methyl-1-piperazinyl-2(3H)-benzoxazolone or its monohydrochloride the preferred pH ranges from 5.0 to 6.0. The most preferred pH for said compound is 5.5.

During the delivery period, the current may be caused to flow by applying a constant, pulsed, or alternating voltage/current. Alternatively the current may be caused to increase during the delivery period in order to titrate an increasing concentration of the compound of formula (I).

The voltage charged in the current application step is selected in th e range of voltage that does not injure the skin of a living body and that does not disadvantage the rate of the transdermal absorption of the active compound. The range is e.g., from about 0.1 to 40 V, preferably from 0.5 to 20 V and more preferably from about 1 to 10 V.

The pulsed or alternating voltage may have a frequen cy from about 0.01 Hz to about 200 kHz, preferably about 100 Hz to 100 kHz and most preferably about 5 to 80 kHz, using substantially any type of waveform shape, including sine, square, triangular, sawtooth, rectangular, etc. In addition, the pulsed or alternating voltage may be applied on a duty cycle less than 100%.

The current density is generally about 0.001 to 1.0 mA/cm², preferably about 0.005 to 0.8 mA/cm² and more preferably about 0.025 to 0.5 mA/cm².

The drug reservoir contains the drug and electrolyte with as the vehicle or carrier either an aqueous solution or a (hydro)gel. The reservoir gel may be comprised of water soluble polymers or hydrogels. In principle any gel can be used, as long as it does not adversely affect the skin (corrosion and irritation) and has suitable properties, such as good skin contact (adhesiveness) and electroconductive property. Typical examples are agar, agarose, polyvinyl alcohol, or crosslinked hydrogels, such as Hydroxymethylpropylcellulose (HPMC), Methylcellulose (MC), Hydoxyethylcellulose (HEC), Carboxymethylcellulose (CMC) and Polyvinylpyrrolidone (PVP) and Polyvinyl Acetate Phtalate (PVAP).

Suitable skin penetration enhancers include those well known in the art, for example, C₂-C₄ alcohols such as ethanol and isopropanol; surfactants, e.g. anionic surfactants such as salts of fatty acids of 5 to 30 carbon atoms, e.g. sodium lauryl sulphate and other sulphate salts of fatty acids, cationic surfactants such as alkylamines of 8 to 22 carbon atoms, e.g. oleylamine, and nonionic surfactants such as polysorbates and poloxamers; aliphatic monohydric alcohols of 8 to 22 carbon atoms such as decanol, lauryl alcohol, myristyl alcohol, palmityl alcohol, linolenyl alcohol and oleyl alcohol; fatty acids of 5 to 30 carbon atoms such as oleic acid, stearic acid, linoleic acid, palmitic acid, myristic acid, lauric acid and capric acid and their esters such as ethyl caprylate, isopropyl myristate, methyl laurate, hexamethylene palmitate, glyceryl monolaurate, polypropylene glycol monolaurate and polyethylene glycol monolaurate; salicylic acid and its derivatives; alkyl methyl sulfoxides such as decyl methyl sulfoxide and dimethyl sulfoxide; 1 -substituted azacycloalkan-2-ones such as 1-dodecylazacyclo-heptan-2-one (AZONE®); amides such as octylamide, oleicamide, hexamethylene lauramide, lauric diethanolamide, polyethylene glycol 3 - lauramide, N,N-diethyl-m-toluamide and crotamiton; and any other compounds compatible with compounds of the general formula (I) and the patches and having transdermal permeation enhancing activity.

In an alternative embodiment the carrier or vehicle is separated from the skin by a membrane. This membrane should preferably have a low re sistance against the electric current, should not substantially raise the barrier against the transport of the active compound and should be able to contain the carrier within the patch during storage and transport. A low resistance against the electronic current is defined as 20% of the resistance of the skin. The barrier against transport of the active compound is not substantially raised by the membrane when the flux of active compound in the membrane containing patch is more than 75% compared with the patch not containing a membrane. Examples of membranes that can be used are e.g. the membranes having low electrical resistance as disclosed in D.F. Stamiatialis et al., J. Controlled Release 2002, 81, 335-345, such a the membranes CT-10 kDA, CT-20 kDA, PES-30 kDA and PSf-100 kDA of Sartorius, Dialysis-5 kDA of Diachema, CA-10 kDa, CA-25 kDa, CA-50 kD and CA-100 kDa of Amika and NF-PES-10 and NF-CA-30 of Nadir Filtration.

The iontophoretic systems used to practice the subject invention may include devices and/or components selected from a wide variety of commercially available devices or components and/or from a wide range of methods and materials such as taught, for example, by patents and publications relating to such iontophoretic systems. In particular, the iontophoretic transdermal system may comprise an iontophoretic device such as is available from The Alza corporation of Mountain View, California, U.S.A. (E -trans® Transdermal Technology), Birch Point Medical Inc. of St. Paul, Minnesota U.S.A. (e.g. IontoPatch™ working according to the Wearable Electronic Disposable Delivery (WEDD™) technology), lomed of Salt Lake City, Utah, U.S.A. (e.g. IOMED™ Phoresor devices using IOGEL®, TransQ®Flex, TransQ®E, TransQ®1&2 or Numby Stuff® electrodes and the GelSponge® containment medium), or a device such as manufactured by Vyteris of Fair Lawn, New Jersey, U.S.A. (Active Transdermal system) or a device such as manufactured by Empi of St. Paul, Minnesota (e.g. Empi DUPEL™), or a device known as the LECTRO™ Patch, manufactured by General Medical Device Corp. of Los Angeles, California.

The electrodes may be comprised of reactive or non-reactive electrodes. Examples of reactive electrodes are those made from metal salts, such as silver chloride or materials described in US 4,752,285. The silver chloride electrodes can be prepared based on the knowledge of a person skilled in the art or are available from lomed. Alternative reactive electrodes can be made from a combination of ion-exchange resins, exemplified by electrodes available from Empi. Examples of non-reactive electrodes are those made from metals such as gold or platinum, or from carbon particles dispersed in polymeric matrices such as one used in the LECTRO™ Patch. Adhesives used to fix the iontophoretic patch to the skin may be comprised of pressure sensitive adhesives used in passive transdermal delivery systems, such those derived from silicone or acrylic polymers, or those derived from rubbers such as polyisobutylene. A combination of pressure sensitive and conductive adhesives can also be used, such as those described EPA 0542294.

In the drug reservoir, the concentration of the drug may typically range from 0.1 to about 90 mg/ml, A preferred concentration for 7-(4-methyl-1-piperazinyl-2(3H)-benzoxazolone or its monohydrochloride is about 10 to about 75 mg/ml, and more preferably about 20 to about 55 mg/ml.

Additionally, the drug reservoir of the iontophoretic system may include additives that are well known and conventional in the iontophoresis art. Such additives include, for example, antimicrobial agents, preservatives, antioxidants, penetration enhancers and buffers.

The representative unit dosage that may typically be delivered during a single delivery period may vary in amount from about 0. 05 mg to about 100 mg. A preferred unit dosage for 7-(4-methyl-1-piperazinyl-2(3H)-benzoxazolone or its monohydrochloride is about 0.05 to about 60 mg, more preferably about 0.05 to about 30 mg.

The unit dosage that is delivered may be determined on the basis of a wide range of factors, including the compound, condition, age, body weight, clearance, etc.

The flux rate of delivery through the skin of the compound with formula I is typically between 50 µg and 4000µg per hour.

Often, the iontophoretic delivery method of pharmaceutical compounds comprises a drug delivery treatment protocol that includes periodically applying an iontophoretic transdermal patch at intervals that may be as frequent as twice daily or as infrequent as once a week or once a month. Typically, in what is herein referred to as a single treatment step, the patch is applied, the drug is iontophoretically delivered and the patch is then removed, with another patch being applied again, when the next treatment step is due to be carried out. Although the absolute quantity of the drug delivered may vary substantially, a unit dosage is herein defined to be that quantity of drug, however large or small, that is delivered during a single treatment step by a single patch application at an individual site.

During the single treatment step the drug may be delivered constantly or during defined intervals. The intervals may typically range from about 10 minutes to 24 hours. In some cases it may be advantageous to omit delivery during a part of the day and night cycle.

Upon starting the delivery of the drug it is often desirable to have a linear or stepwise increase of the drug over a certain time starting with a low amount of drug up to the normal maintenance dose, which time is also referred to as titration time. The period for titration is typically between 3 and 42 days, preferably between 7 and 21 days and most preferred around 14 days. The iontophoretic delivery method according to the present invention is especially useful for such a linear or stepwise increase of the drug administration as the administered amount of drug can be regulated precisely by linear or stepwise increase of the current density.

The iontophoretic system may be comprised of still other methods and materials, such as described in WO 92/17239, EPA 0547482 and US 4,764,164.

Generally, the transport area of the patch ranges from about 1.0 to about 30 cm², preferably about 2 to about 15 cm², and more preferably about 5 to about 10 cm².

In an alternative embodiment of the invention the drug reservoir of the iontophoretic system is delivered empty to the user and the reservoir is filled just before or after application of the system to the skin. When using this embodiment the iontophoretic system is combined in a kit with a number of cartridges containing the compound of general formula I as defined above, including a salt or prodrug therof, or a composition thereof and optionally a pharmaceutically acceptable electrolyte. This kit, which may also be defined as a starter kit, may contain one or more cartridges with the active compound. The amount of cartridges in one kit is typically 7 to 91 and preferably 14 to 28. The compound and the optional electrolyte may be in the form of a solid crystalline, amorphous or lyophilized material which material has to be dissolved in water before filling of the reservoir of the iontophoretic device, or in the form of a solution ready for use. The iontophoretic system may be refilled with a fresh solution every 3-48 hours, but preferably is fill ed once every 24 hours. In a second kit which is intended for further use, as long as the iontophoretic system is properly working, only a one or more of cartridges comprising the compound of general formula I as defined above, including a salt or prodrug thereof, or a composition thereof and optionally a pharmaceutically acceptable electrolyte are present. In the framework of the present embodiment of the invention a unit dose is defined as that quantity of drug that is present in a cartridge as mentioned.

The following examples are only intended to further illustrate the invention, in more detail, and therefore these examples are not deemed to restrict the scope of the invention in any way.

### EXAMPLES

### Example 1. General methods.

### Stratum Corneum isolation

Human Stratum Corneum (HSC) was prepared from dermatomed healthy human skin. Within 24 hours after surgical removal of the human skin (abdominal or breast), residual subcutaneous fat was removed. To avoid interference with contaminating subcutaneous fat, the skin surface was carefully wiped with a tissue paper soaked in 70% ethanol. The skin was dermatomed to a thickness of about 300 µm using a Padgett Electro Dermatome Model B (Kansas City, USA). It was then incubated with the dermal side on Whatman paper soaked in a solution of 0.1% trypsin in PBS overnight at 4 °C and subsequently for 1 hour at 37 °C. Then HSC was peeled off from the underlying epidermis and dermis. Remaining trypsin activity was blocked by bathing HSC in a 0.1% trypsin inhibitor solution in PBS, pH 7.4. HSC was washed several times in water and stored in a silica gel containing desiccator in a N₂ environment to inhibit oxidation of lipids.

### Active compound synthesis

The active compound was synthesized as described in WO00/29397.

### Solutions in iontophoresis experiments

The active compound as a salt was dissolved in 10 mM sodium citrate solution. The pH was adjusted to pH 5.5 with 10 mM citric acid.

### Iontophoresis

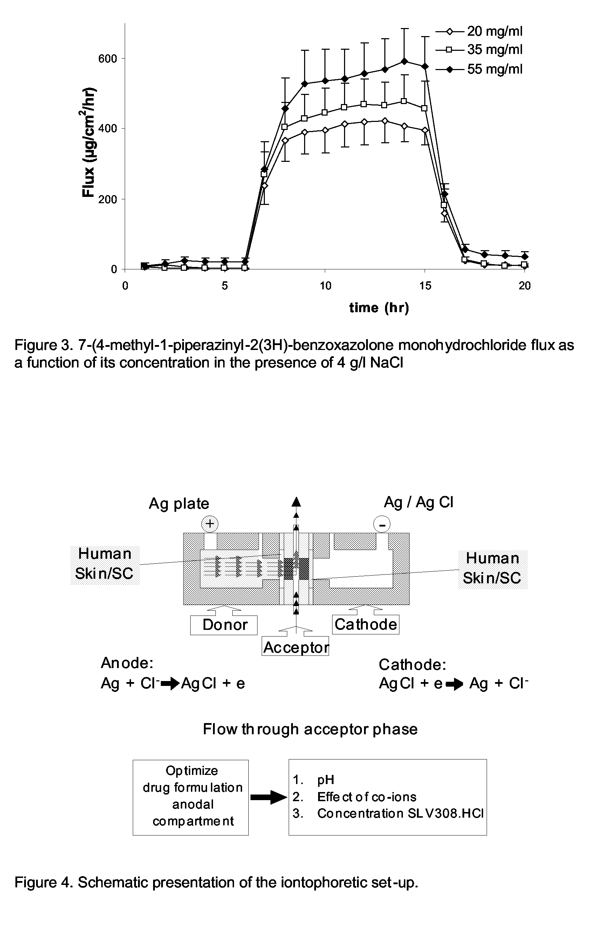

Iontophoresis was performed using 9-channel computer controlled power suppl y to provide constant current (Electronics Department, Gorlaeus Laboratories, Leiden University, The Netherlands. Alternatively the commercial available Power Supply PCT-MK1 of Moor Instruments, UK can be used.. A silver plate electrode was used as an anode (e.g. Silver foil >99.99% pure, 1.0 mm thick (Aldrich article nr. 36,943-8), 5 cm long, 3 mm wide) and a silver/silver chloride electrode (prepared by repeatedly (2 or 3 times) dipping silver wire (>99.99% pure, ∅ 1.0 mm (Aldrich art. Nr. 26,559-4), bended at the tip to produce a small projection (approx. 3 mm) at a right angle to the vertical electrode shaft in melted solverchloride powder (>99.999% pure, Aldrich art. Nr. 20,438-2) as a cathode. (alternatively the silver plate and silver/silver chloride electrodes can be prepared according to chapter 3.4.3. of Ajay K. Banga, Electrically Assisted Transdermal and Topical Drug Dilivery, Taylor and Francis Group Ltd., London UK, 1998, ISBN 0-7484-0687-5. or can be purchased from a commercial supplier such as lomed)
All diffusion experiments were carried out at a constant current density of 0.5 mA/cm², using three chambers continuous flow through diffusion cells at room temperature. The diffusion set up consisted of a peristaltic pump, a fraction collector and 8 diffusion cells (for diffusion cell see figure 4). Stratum corneum was used for all diffusion studies. Human stratum corneum was hydrated for two hours in PBS pH 7.4 prior to mounting in the cells. Two pieces of stratum corneum were placed between the anodal and acceptor side, and between acceptor and cathodal side, with the apical side facing the anodal and cathodal compartments. Dialysis membrane (cut off 5,000 D) was used as supporting membrane for the stratum corneum. Parafilm rings were added for making a tight connection between the compartments. The temperature of the acceptor chamber was 37°C. The flow of PBS through the acceptor chamber was kept approximately constant for each cell during the experiment: 6 - 8 ml per hour. After six hours of passive diffusion, the current was switched on. The current was switched off at t = 15 h. During a period of another 5 hours (post iontophoretic period) passive diffusion post iontophoresis was carried out. During iontophoresis, the current density was 0.5 mA/cm². The total resistance of the stratum corneum sheets was monitored during the experiment with two additional silver electrodes. A very low resistance is indicative of leakage of the stratum corneum in a cell. When this was observed, the diffusion dat a obtained were discarded. Al conditions were repeated at least 3 times. The number of skin donors used for each condition was at least 3.

### HPLC analysis

7-(4-methyl-1-piperazinyl-2(3H)-benzoxazolone was analyzed using HPLC with UV detection (Waters Chromatography, Etten Leur, The Netherlands). A Chromsep SS column was used (250*3 mm L*i.d.) thermostatted at 30 °C. The mobile phase consisted of acetonitrile/methanol/ 0.7 g/I ammonium acetate buffer at pH 5.6 (12/6/82 v/v) and was used at 0.5 ml/min. The detection wavelength was 215 nm.
No oxidation or degradation products of the compound were observed in the chromatograms of the sample solutions.

### Example 2. Iontophoresis of 7-(4-methyl-1-piperazinyl-2(3H)-benzoxazolone monohydrochloride with varying active substance concentration

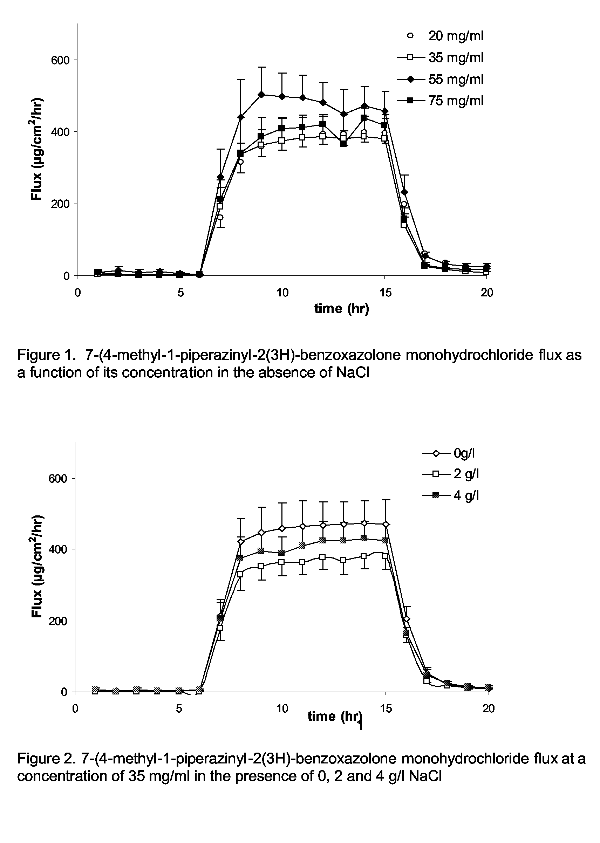

A solution of 75mg/ml of 7-(4-methyl-1-piperazinyl-2(3H)-benzoxazolone monohydrochloride in citrate buffer was prepared (This is 85% of the maximum solubility of 7-(4-methyl-1-piperazinyl-2(3H)-benzoxazolone monohydrochloride in citrate buffer at pH 5.5). From this solution further dilutions were made in citrate buffer pH 5.5. The concentrations tested were: 20 mg/ml, 35 mg/ml, 55 mg/ml and 75 mg/ml.
As can be observed in figure 1, after switching on the current, there is a steep increase in 7-(4-methyl-1-piperazinyl-2(3H)-benzoxazolone flux. During the iontophoresis period, the 7-(4-methyl-1-piperazinyl-2(3H)-benzoxazolone fluxes observed were extremely high. The mean transport during the iontophoretic period was 394 ± 26, 383 ± 42, 459 ± 59, 418 ± 31 µg/hr/cm² for the donor concentrations of 20, 35, 55, 75 mg/ml respectively. There was no significant difference between these values as tested by one way ANOVA (p-value between all groups > 0.05)
The pH of the donor solution did not change more than 0.2 pH units during the experiment.

### Example 3. Iontophoresis of 7-(4-methyl-1-piperazinyl-2(3H)-benzoxazolone monohydrochloride with varying active electrolyte concentration

(7-(4-methyl-1-piperazinyl-2(3H)-benzoxazolone monohydrochloride was dissolved in 10 mM sodium citrate solution. The pH was adjusted to pH 5.5 with 10 mM citric acid. Sodium chloride was added resulting in solutions of either 0, 2 or 4 mg/ml NaCl. The (7-(4-methyl-1-piperazinyl-2(3H)-benzoxazolone monohydrochloride concentration was kept constant, namely 35 mg/ml. At 4 mg/ml NaCl, the selected (7-(4-methyl-1-piperazinyl-2(3H)-benzoxazolone monohydrochloride concentration is 80% of its maximum solubility. The solubility of (7-(4-methyl-1-piperazinyl-2(3H)-benzoxazolone monohydrochloride increases with reducing NaCl concentration.

Figure 2 illustrates that, after switching on the current, there is a steep increase in (7-(4-methyl-1-piperazinyl-2(3H)-benzoxazolone flux. During the iontophoresis period, the (7-(4-methyl-1-piperazinyl-2(3H)-benzoxazolone fluxes observed were extremely high. The mean transport during the iontophoretic period was 471 ± 65, 377 ± 37 and 424 ± 50 µg/hr/cm² (averages ± s.e.m.) for the sodium chloride concentrations 0, 2, 4 mg/ml, respectively. There was no significant difference between these values as tested by one way ANOVA (p-value between all groups > 0.05). The pH of the donor solution did not change more than 0.2 pH units during the experiment.
The strong increase and decrease during switching on and off the current indicates that a large variation in transport can be achieved by iontophoresis.

### Example 4. Iontophoresis of (7-(4-methyl-1-piperazinyl-2(3H)-benzoxazolone monohydrochloride with varying active substance concentration in the presence of 4 g/l NaCl.

A solution of 55mg/ml of (7-(4-methyl-1-piperazinyl-2(3H)-benzoxazolone monohydrochloride in citrate buffer was prepared (This is 85% of the maximum solubility of (7-(4-methyl-1-piperazinyl-2(3H)-benzoxazolone monohydrochloride in citrate buffer at pH 5.5 in the presence of 4 g/I NaCl). From this solution further dilutions were made in citrate buffer pH 5.5. The concentrations tested were: 20 mg/ml, 35 mg/ml, 55 mg/ml and NaCl was added in amount to yield a concentration of 4 g/l.
Figure 3 shows that in the presence of NaCl the iontophoretic flux of (7-(4-methyl-1-piperazinyl-2(3H)-benzoxazolone monohydrochloride was slightly dependent on its concentration flux. The fluxes were 409 ± 47, 467 ± 74 and 580 ± 87 µg/hr/cm² for the donor concentrations of 20, 35 and 55 mg/ml respectively (averages ± s.e.m.) . However, the trend appeared to be not statistically significant as tested by one way ANOVA (p-value between all groups > 0.05).
The pH of the donor solution did not change more than 0.2 pH units during the experiment.

## Claims

1. The use of a compound of the general formula 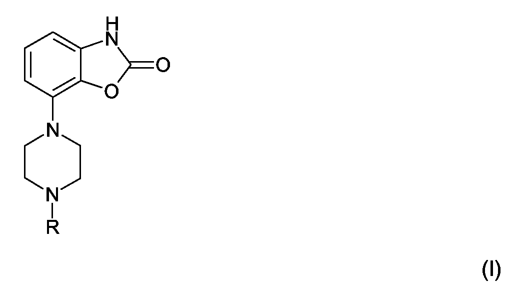 wherein R is methyl, ethyl, ethyl substituted with one or more fluorine atoms, or cycloalkyl-(C₃ - C ₇) - methyl optionally substituted with one or more fluorine atoms or benzyl optionally substituted with a group selected from phenyl, furanyl and thienyl which groups are optionally substituted with with 1 -3 substituents from the group hydroxy, halogen, C₁₋₄-alkoxy, C₁₋₄-alkyl, cyano, aminocarbonyl, mono- or di-C₁₋₄-alkylaminocarbonyl; and pharmaceutically acceptable salts and prodrugs thereof, for the manufacture of a composition suitable for use in a patch for transdermal administration by iontophoresis, wherein said composition comprises the compound of formula I and optionally a pharmaceutically acceptable electrolyte.

2. The use of a compound of formula I as claimed in claim 1, for the manufacture of a patch suitable for transdermal administration by iontophoresis, wherein said transdermal patch has a reservoir containing the compound of formula I or a composition thereof and optionally a pharmaceutically acceptable electrolyte.

3. The use according to claims 1 or 2, wherein the compound of formula I and the optional electrolyte are dissolved in a vehicle or carrier consisting of an aqueous solution or a gel.

4. The use according to claim 2 or 3, wherein the patch additionally contains a membrane which separates the vehicle or carrier from the skin when applied for transdermal administration by iontophoresis.

5. The use according to claims 2-4, **characterized in that** said patch is able to deliver a constant current during the current application step in the transdermal administration by iontophoresis.

6. The use according to claims 2-4, **characterized in that** said patch is able to deliver a variable current during the current application step in the transdermal administration by iontophoresis.

7. The use according to claim 5 or 6, **characterized in that** said patch is able to deliver an increasing current during the current application step in the transdermal administration by iontophoresis.

8. The use according to claims 2-6, **characterized in that** said patch is able to deliver a current density at a level from about 0.001 to about 1.0 mA/cm².

9. The use accord ing to claims 2-6, **characterized in that** the fluxrate through the skin of the compound of formula I is between 50 µg and 4000 µg per hour.

10. The use according to claims 1-9, wherein the compound concentration in the solution is 0.1 to 90 mg/ml.

11. The use according to claims 1-10, wherein the pH of the solution is between 3.0 and 7.5.

12. The use according to claim 2-11, **characterized in that** said patch is able to deliver a unit dosage of about 0.1 mg to about 200 mg of the compound of formula I through the skin during the delivery step.

13. The use according to claims 2-12, **characterized in that** said patch is able to deliver the unit dosage over a time period of about 10 minutes to about 48 hours.

14. The use according to claims 1-13, wherein the compound of formula I is 7-(4-methyl-1-piperazinyl-2(3H)-benzoxazolone or a pharmaceutically acceptable salt thereof.

15. The use according to claim 14, wherein the compound of formula I is 7-(4-methyl-1-piperazinyl-2(3H)-benzoxazolone) monohydrochloride.

16. The use according to claims 1-13, wherein the compound of formula I is 7-(4-benzyl-1-piperazinyl-2(3H)-benzoxazolone or a pharmaceutically acceptable salt thereof.

17. The use according to claim 16, wherein the compound of formula I is 7-(4-benzyl-1-piperazinyl-2(3H)-benzoxazolone monomesylate.

18. An iontophoretic system for the delivery of a compound through skin, comprising(a) a transdermal delivery device attachable to the skin, the device including a first electrode and a second electrode, and a reservoir containing a compound of general formula I or a composition thereof and optionally a pharmaceutically acceptable electrolyte in electrical communication with the first and second electrodes and (b) a electrical power source connected to the first and second electrodes and (c) optionally a membrane closing the reservoir during transport, storage and/or application.

19. The iontophoretic system of claim 18, wherein the compound is 7-(4-methyl-1-piperazinyl-2(3H)-benzoxazolone or a pharmaceutically acceptable salt thereof.

20. The iontophoretic system of claim 19, wherein the compound is 7-(4-benzyl-1-piperazinyl-2(3H)-benzoxazolone or a pharmaceutically acceptable salt thereof.

21. The iontophoretic system of claims 18-20, wherein reservoir contains the pharmaceutically acceptable electrolyte and compound of general formula I or a composition thereof in a solution having a pH ranging from about 3.5 to about 7.5.

22. The iontophoretic system of claims 18-21, wherein the compound of general formula I or a composition thereof is present in the reservoir in a solution at a concentration of about 0.1 to about 90 mg/ml.

23. A kit comprising
(1) an iontophoretic system for the delivery of a compound through skin, comprising (a) a transdermal delivery device attachable to the skin, the device including a first electrode and a second electrode, and a reservoir able to contain a composition of an active compound and (b) a electrical power source connected to the first and second electrodes and (c) optionally a membrane closing the reservoir during transport, sto rage and/or application
(2) one or more cartridges comprising a compound of general formula I as claimed in claim 1, including a salt or prodrug therof, or a composition thereof and optionally a pharmaceutically acceptable electrolyte.

24. A kit comprising one or more cartridges comprising a compound of general formula I as claimed in claim 1, including a salt or prodrug thereof, or a composition thereof and optionally a pharmaceutically acceptable electrolyte.

25. The kit according to claims 23-24, wherein the compound is 7-(4-methyl-1-piperazinyl-2(3H)-benzoxazolone or a pharmaceutically acceptable salt thereof.

26. The kit according to claims 23-24, wherein the compound is 7-(4-benzyl-1-piperazinyl-2(3H)-benzoxazolone or a pharmaceutically acceptable salt thereof.

27. The kit according to claims 23-26, wherein the cartridge comprises the pharmaceutically acceptable electrolyte and compound of general formula I or a composition thereof in a solution having a pH ranging from about 3.5 to about 7.5.

28. The kit according to claims 23-27, wherein the cartridge comprises the compound of general formula I or a composition in a solution at a concentration of about 0.1 to about 90 mg/ml.
